# EUROPÄISCHE PATENTSCHRIFT

(11) **EP 2 416 200 B1**
(45) Veröffentlichungstag und Bekanntmachung des Hinweises auf die Patenterteilung: **08.01.2020**
(21) Anmeldenummer: 11176122.7
(22) Anmeldetag: 01.08.2011
(51) Int. Cl.: G02B 26/10, A61B 1/00, G02B 23/24

(54) **Endoskop mit einstellbarer Blickrichtung**
Endoscope with adjustable viewing direction
Endoscope à direction d'observation ajustable

(30) Priorität: 04.08.2010 DE 102010033425
(43) Veröffentlichungstag der Anmeldung: 08.02.2012
(73) Patentinhaber: Karl Storz SE & Co. KG, 78532 Tuttlingen (DE)
(72) Erfinder: Weiger, Ulrich, 78532 Tuttlingen (DE); Leibinger, Doris, 78532 Tuttlingen (DE)

(56) Entgegenhaltungen:
- WO-A1-2004/066614
- GB-A- 2 354 836
- US-A- 5 912 764
- US-A1- 2003 092 966

## Beschreibung

Die vorliegende Erfindung bezieht sich auf ein Endoskop mit einstellbarer Blickrichtung und ein Verfahren zum Erfassen eines Bilds mittels eines Endoskops.

Neben Endoskopen für medizinische und nicht-medizinische technische Anwendungen, deren Blickrichtung parallel zur Längsachse des Schafts des Endoskops ist, wurden bereits früh Endoskope mit anderen feststehenden Blickrichtungen entwickelt. Mit der Blickrichtung eines Endoskops ist hier und im Folgenden immer die Richtung vom distalen Ende des Endoskops aus gemeint, in der ein Gegenstand liegt, der in der Mitte des mittels des Endoskops erfassten Bilds erscheint. Bei vielen Anwendungen ist jedoch eine feste Blickrichtung nachteilig. Im ungünstigsten Fall muss beispielsweise während eines medizinischen Eingriffs mehrfach das Endoskop gewechselt werden. In solchen Fällen ist die Verwendung eines Endoskops mit einer in situ einstellbaren bzw. verstellbaren Blickrichtung vorteilhaft.

Ein Schwenkprismenendoskop weist am distalen Ende ein schwenkbares Prisma auf, an dessen Grenzflächen in das Endoskop fallendes Licht gebrochen und reflektiert wird, bevor es beispielsweise mittels einer Stablinsenoptik zum proximalen Ende des Endoskops geleitet wird. Durch Schwenken des Prismas um eine Achse senkrecht zur Längsachse des Schafts des Endoskops ist die Blickrichtung einstellbar.

Bei herkömmlichen Schwenkprismenendoskopen sind Blickrichtungsbereich, Bildfeldwinkel, Helligkeit und Bildqualität oft unbefriedigend. Wenn der Blickrichtungsbereich eine Blickrichtung parallel zur Achse des Schafts des Endoskops (0 Grad) umfassen soll, war lange nur ein kleiner Blickrichtungsbereich realisierbar. Ein großer Blickrichtungsbereich, zwischen dessen extremen Blickrichtungen 120 Grad oder mehr liegen, war bisher nicht realisierbar.

In der DE 600 15 375 T2 ist ein Endoskop mit variabler Blickrichtung beschrieben, das zwei Rechtwinkel-Prismen umfasst. Eines der Rechtwinkel-Prismen ist um eine Rotationsachse, welche orthogonal zur Längsachse des Endoskops ist und diese schneidet, rotierbar.

US 2003/0092966 A1 offenbart ein Endoskop mit einstellbarer Blickrichtung und schwenkbaren Prismen mit planen Flächen und ohne weitere Ausnehmungen.

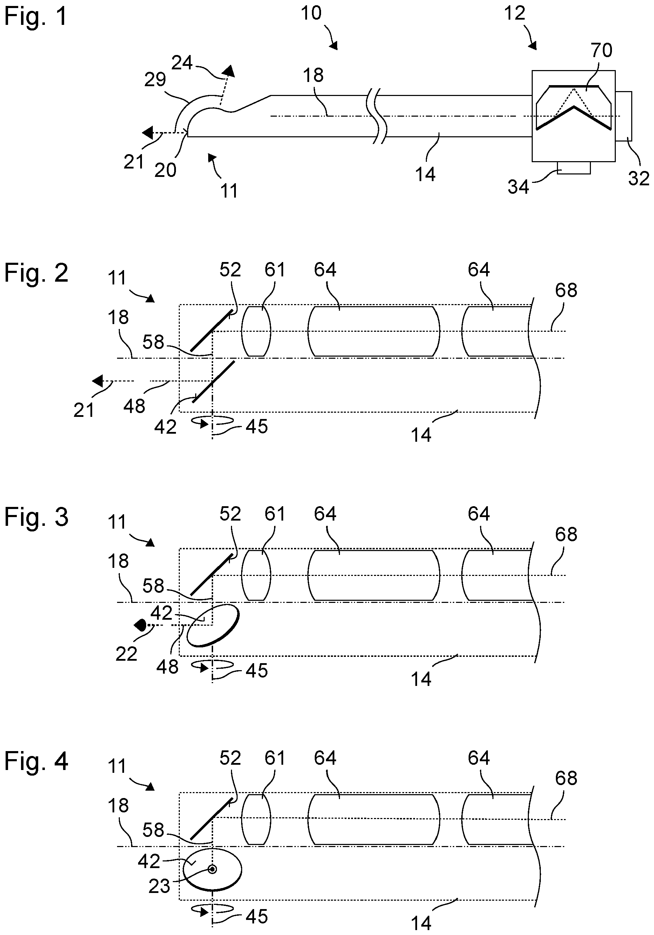

WO 2004/066614 A1 offenbart einen Suchkopf für eine Zielverfolgungseinrichtung, wobei ein schwenkbares Prisma in einer Ausnehmung eines feststehenden Prismas liegt (Abb. 2) oder umgekehrt (Abb. 3). Beide Prismen weisen plane Flächen auf.

GB 2354836 A offenbart ebenfalls eine Anordnung mit schwenkbarem Prisma.

US5912764 offenbart eine feststehende Prismenanordnung mit gekrümmten Flächen.

Eine Aufgabe der vorliegenden Erfindung besteht darin, ein verbessertes Endoskop mit einstellbarer Blickrichtung zu schaffen.

Diese Aufgabe wird durch die Gegenstände der unabhängigen Ansprüche gelöst. Weiterbildungen sind in den abhängigen Ansprüchen angegeben.

Ein Endoskop mit einstellbarer Blickrichtung umfasst ein schwenkbares Prisma zum Umlenken von Licht, das zum Einstellen der Blickrichtung um eine Schwenkachse schwenkbar ist, und ein feststehendes Prisma zum Umlenken von Licht, das von dem schwenkbaren Prisma umgelenkt ist, in eine Richtung parallel zur Längsachse des Endoskops, wobei zumindest entweder eine Lichtaustrittsfläche des schwenkbaren Prismas in einer Ausnehmung des feststehenden Prismas oder eine Lichteintrittsfläche des feststehenden Prismas in einer Ausnehmung des schwenkbaren Prismas angeordnet ist.

Eine Ausnehmung in einem Prisma umfasst insbesondere einen konkaven Abschnitt der Oberfläche des Prismas, wobei der konkave Abschnitt ebene oder lokal konvexe Teilbereiche aufweisen kann. Ein Abschnitt der Oberfläche des Prismas wird insbesondere als konkav bezeichnet, wenn die Oberfläche in einem Querschnitt entlang einer Schnittebene konkav im oben genannten Sinn ist, wobei die Schnittebene insbesondere die optische Achse distal und proximal der reflektierenden Fläche umfasst.

Als konvex wird hier und im Folgenden ein Körper bezeichnet, dessen Oberfläche an jeder Stelle nach außen gewölbt oder eben ist. Ein Abschnitt einer Oberfläche eines Körpers wird als konvex bezeichnet, wenn der Abschnitt der Oberfläche nach außen gewölbt ist. Ein Abschnitt einer Oberfläche eines Körpers wird als konkav bezeichnet, wenn der Abschnitt der Oberfläche nach innen gewölbt ist. Ein Abschnitt eines Rands einer Querschnittsfläche wird als konvex bezeichnet, wenn der Abschnitt des Rands nach außen gewölbt ist, und als konkav, wenn er nach innen gewölbt ist.

Die optische Achse ist im Fall eines rotationssymmetrischen optischen System die Symmetrieachse des optischen Systems. Soweit hier beschriebene Endoskope rotationssymmetrische optische Komponenten aufweisen, beispielsweise ein Stablinsensystem, ist die optische Achse durch deren Symmetrieachse jeweils eindeutig gegeben. Gegebenenfalls wird eine Rotationssymmetrie jedoch jedenfalls durch die Prismen gebrochen. Selbst wenn die Lichtaustrittsfläche des feststehenden Prismas ebenfalls rotationssymmetrisch zur optischen Achse eines lichtstromabwärts sich anschließenden Stablinsensystems ist, bricht das Prisma aufgrund seiner Licht umlenkenden Eigenschaft zwangsläufig die Rotationssymmetrie.

Wenn ein hier beschriebenes Endoskop rotationssymmetrische optische Komponenten aufweist, wird dort, wo die Rotationssymmetrie, insbesondere durch die Prismen, gebrochen ist, als optische Achse diejenige Linie bezeichnet, in der im Bild der geometrischen Optik ein Lichtstrahl liegt, der in den rotationssymmetrischen optischen Komponenten in deren Symmetrieachse liegt. Insbesondere fällt ein Lichtstrahl, der entlang der optischen Achse verläuft, auf die Mitte eines Bildsensors des Endoskops oder einer mit dem Endoskop gekoppelten Kamera.

Wenn ein hier beschriebenes Endoskop keine rotationssymmetrischen optischen Komponenten aufweist, oder die oben gegebene Definition des Begriffs der optischen Achse aus anderen Gründen nicht anwendbar ist, wird als optische Achse insbesondere eine Linie bezeichnet, entlang derer im Bild der geometrischen Optik ein Lichtstrahl verläuft, der zu einem Bildpunkt in der Mitte eines mittels des Endoskops erfassten Bilds liegt. Das mittels des Endoskops erfasste Bild kann durch eine Kamera oder durch das menschliche Auge an einem Okular des Endoskops erfasst werden. Insbesondere verläuft die optische Achse in der Mitte oder nahe der Mitte der optischen Komponenten des Endoskops.

Abweichend von der Situation in einem rein rotationssymmetrischen optischen System weist die optische Achse also vorliegend jedenfalls nicht die Gestalt einer Geraden auf. Zumindest soweit der Brechungsindex sich nur an Grenzflächen zwischen zwei Medien sprunghaft und nicht auch innerhalb eines Mediums kontinuierlich ändert, ist die optische Achse aber abschnittsweise gerade. Beispielsweise weist die optische Achse drei gerade Abschnitte auf, nämlich einen distalen Abschnitt distal bzw. lichtstromaufwärts des schwenkbaren Prismas, einen proximalen Abschnitt proximal bzw. lichtstromabwärts des feststehenden Prismas und einen mittleren Abschnitt zwischen den beiden Prismen. Zumindest wenn die Lichteintrittflächen und die Lichtaustrittflächen der Prismen jeweils rotationssymmetrisch zur optischen Achse sind, weist die optische Achse genau drei gerade Abschnitte auf, die an reflektierenden Flächen der Prismen aneinander grenzen.

Bei Anordnung der Lichtaustrittsfläche des schwenkbaren Prismas in einer Ausnehmung des feststehenden Prismas bildet die Lichteintrittsfläche des feststehenden Prismas einen innerhalb der Ausnehmung liegenden Teil der Oberfläche des feststehenden Prismas. Bei Anordnung der Lichteintrittsfläche des feststehenden Prismas in einer Ausnehmung des schwenkbaren Prismas bildet die Lichtaustrittsfläche des schwenkbaren Prismas einen innerhalb der Ausnehmung liegenden Teil der Oberfläche des schwenkbaren Prismas.

Die beschriebene Anordnung der Lichtaustrittsfläche des schwenkbaren Prismas und der Lichteintrittsfläche des feststehenden Prismas in bzw. an einer Ausnehmung des feststehenden Prismas und/oder der Lichtaustrittsfläche des schwenkbaren Prismas und der Lichteintrittsfläche des feststehenden Prismas in bzw. an einer Ausnehmung des schwenkbaren Prismas kann eine besonders kompakte Bauweise beider Prismen ermöglichen. Damit können ein besonders geringer Abstand zwischen den reflektierenden Flächen der Prismen und ein besonders geringer seitlicher Versatz zwischen einem distalen Abschnitt der optischen Achse distal des schwenkbaren Prismas und dem proximalen Abschnitt der optischen Achse proximal des feststehenden Prismas realisierbar sein. Dies gilt insbesondere, wenn die reflektierenden Flächen der der Prismen unterschiedlich groß sind, und/oder wenn das Lichtbündels im Bereich des schwenkbaren Prismas und/oder im Bereich des feststehenden Prismas eine Einschnürung aufweist. Durch einen geringen seitlichen Versatz können bei gegebenem Querschnitt des Schafts des Endoskops besonders große Querschnitte von Stablinsen oder anderen Einrichtungen zum Übertragen von Licht im Schaft des Endoskops bei gleichzeitig zentrischer oder nicht all zu exzentrischer Anordnung des Lichteintritts am distalen Ende des Endoskops ermöglicht werden.

Das schwenkbare Prisma ist insbesondere ausgebildet und angeordnet, um Licht aus der Blickrichtung in eine Richtung umzulenken, die im Wesentlichen parallel zur Schwenkachse des schwenkbaren Prismas ist. Die Blickrichtung und die Schwenkachse schließen insbesondere einen im Wesentlichen rechten Winkel ein. Da die Blickrichtung insbesondere durch die Richtung des distalen Abschnitts der optischen Achse distal des schwenkbaren Prismas gegeben ist, und da die Schwenkachse insbesondere mit dem mittleren Abschnitt der optischen Achse zwischen den beiden Prismen zusammenfällt, schließen insbesondere der distale Abschnitt der optischen Achse und der mittlere Abschnitt der optischen Achse einen rechten Winkel ein.

Wenn das schwenkbare Prisma eine ebene reflektierende Fläche umfasst, können der Winkel zwischen der Blickrichtung und der Normalen der reflektierenden Fläche und der Winkel zwischen der Normalen der reflektierenden Fläche und der Schwenkachse des schwenkbaren Prismas jeweils 45° betragen. Wenn die reflektierende Fläche des schwenkbaren Prismas gekrümmt ist, kann die vorstehende Aussage für die Flächennormale der reflektierenden Fläche in deren Schnittpunkt mit der optischen Achse gelten. Die Schwenkachse ist insbesondere senkrecht zur Längsachse des Endoskops.

Das feststehende Prisma ist insbesondere ausgebildet und angeordnet, um Licht aus einer Richtung, die im Wesentlichen parallel zur Schwenkachse des schwenkbaren Prismas ist, in eine Richtung umzulenken, die im Wesentlichen parallel zur Längsachse des Endoskops ist. Wenn die Schwenkachse des schwenkbaren Prismas und die Längsachse des Endoskops einen im Wesentlichen rechten Winkel einschließen und das feststehende Prisma eine ebene reflektierende Fläche umfasst, kann eine Flächennormale der reflektierenden Fläche des feststehenden Prismas mit der Schwenkachse des schwenkbaren Prismas und mit der Längsachse des Endoskops jeweils einen Winkel von 45° einschließen. Wenn die reflektierende Fläche des feststehenden Prismas gekrümmt ist, kann die vorstehende Aussage für die Flächennormale der reflektierenden Fläche in deren Schnittpunkt mit der optischen Achse gelten.

Um bei einer gegebenen Einstellung des schwenkbaren Prismas die Blickrichtung auf einem Kegelmantel um die Längsachse des Endoskops zu bewegen, kann das gesamte Endoskop mit dem feststehenden Prisma und der Schwenkachse des schwenkbaren Prismas um seine Längsachse rotiert werden. Alternativ kann das feststehende Prisma zusammen mit der Schwenkachse des schwenkbaren Prismas relativ zu dem Endoskop um die Längsachse des Endoskops rotiert werden. Mit dem Attribut "feststehend" ist somit insbesondere feststehend in Bezug auf die Schwenkachse des schwenkbaren Prismas gemeint. Das feststehende Prisma steht insbesondere auch hinsichtlich des Endoskops fest, kann jedoch, wie beschrieben, alternativ zusammen mit der Schwenkachse des schwenkbaren Prismas um die Längsachse des Endoskops rotierbar sein.

Als Prisma wird ein transparenter Körper mit einer Lichteintrittsfläche, mindestens einer reflektierenden Fläche und einer Lichtaustrittsfläche bezeichnet, der jedoch nicht genau die Gestalt eines Prismas im strengen Sinn der Geometrie aufweisen muss. Die Lichteintrittsfläche, die reflektierende Fläche und die Lichtaustrittsfläche des Prismas können jeweils eben sein, wobei die Normalen der Lichteintrittsfläche, der reflektierenden Fläche und der Lichtaustrittsfläche in einer Ebene liegen bzw. linear abhängig sind. Die Normale der Lichteintrittsfläche, die Normale der reflektierenden Fläche und die Normale der Lichtaustrittsfläche müssen jedoch nicht in einer Ebene liegen.

Ferner können sowohl die Lichteintrittsfläche als auch die reflektierende Fläche und die Lichtaustrittsfläche jeweils gekrümmt sein, um die lichtumlenkende Eigenschaft der reflektierenden Fläche mit einer abbildenden Eigenschaft von einer oder mehreren gekrümmten Grenzflächen in einem Bauelement zu integrieren.

Ferner kann ein Prisma im Sinne der vorliegenden Beschreibung zwei oder mehr reflektierende Flächen aufweisen. Die Reflexion an einer reflektierenden Fläche eines Prismas beruht jeweils insbesondere auf Totalreflexion oder auf einer reflektierenden Beschichtung einer Oberfläche des Prismas.

Mit Ausführungsformen des hier beschriebenen Endoskops können zahlreiche weitere Vorteile realisierbar sein. Insbesondere können extreme Blickrichtungen und extreme Blickrichtungsbereiche, die ohne Weiteres mehr als 180° umfassen können, realisiert werden. Dies gilt insbesondere, wenn, wie erwähnt, die Schwenkachse des schwenkbaren Prismas senkrecht zur Längsachse des Endoskops ist. Ferner können besonders große Sichtfelder realisierbar sein. Insbesondere können ein Sichtfeldwinkel bzw. Bildfeldwinkel von 70° oder mehr realisierbar sein. Ferner können mit Ausführungsformen des hier beschriebenen Endoskops besonders hohe Bildqualitäten und besonders helle Bilder realisierbar sein.

Bei einem Endoskop, wie es hier beschrieben ist, kann eine reflektierende Fläche des feststehenden Prismas größer als eine reflektierende Fläche des schwenkbaren Prismas sein.

Insbesondere beträgt das Verhältnis zwischen dem Inhalt oder dem Durchmesser der reflektierenden Fläche des feststehenden Prismas und dem Inhalt bzw. dem entsprechenden Durchmesser der reflektierenden Fläche des schwenkbaren Prismas mindestens 4:3 oder mindestens 3:2 oder mindestens 2:1.

Alternativ kann bei einem Endoskop, wie es hier beschrieben ist, eine reflektierende Fläche des schwenkbaren Prismas größer als eine reflektierende Fläche des feststehenden Prismas sein.

Insbesondere beträgt das Verhältnis zwischen dem Inhalt oder dem Durchmesser der reflektierenden Fläche des schwenkbaren Prismas und dem Inhalt bzw. dem entsprechenden Durchmesser der reflektierenden Fläche des feststehenden Prismas mindestens 4:3 oder mindestens 3:2 oder mindestens 2:1.

Bei einem Endoskop, wie es hier beschrieben ist, kann ein von dem Endoskop erfasstes Lichtbündel zumindest entweder im Bereich des schwenkbaren Prismas oder im Bereich des feststehenden Prismas eine Einschnürung aufweisen.

Durch die Einschnürung des Lichtbündels im Bereich des schwenkbaren Prismas und/oder im Bereich des feststehenden Prismas kann der von beiden Prismen eingenommene Bauraum stark reduziert werden. Wie nachfolgend anhand der Beschreibung von Ausführungsformen deutlich wird, können dadurch beispielsweise die Querschnitte von Stablinsen zur Übertragung des Lichts zum proximalen Ende des Endoskops vergrößert werden. Durch vergrößerte Querschnitte von Stablinsen können die Helligkeit, der Kontrast und die Schärfe des erfassbaren Bilds verbessert werden.

Sowohl das schwenkbare Prisma als auch das feststehende Prisma kann je eine oder mehrere ebene oder gekrümmte reflektierende Fläche aufweisen.

Bei einem Endoskop, wie es hier beschrieben ist, kann zumindest entweder das schwenkbare Prisma oder das feststehende Prisma eine abbildende Eigenschaft aufweisen.

Bei einem Endoskop, wie es hier beschrieben ist, kann zumindest entweder die Lichteintrittsfläche des schwenkbaren Prismas oder die Lichtaustrittsfläche des feststehenden Prismas gekrümmt sein.

Ferner können sowohl die Lichtaustrittsfläche des schwenkbaren Prismas als auch die Lichteintrittsfläche des feststehenden Prismas jeweils gekrümmt sein. Insbesondere durch gekrümmte Lichteintrittsflächen und/oder Lichtaustrittsflächen sowie durch gekrümmte reflektierende Flächen können die Prismen jeweils abbildende Eigenschaften aufweisen. Dadurch kann unter Umständen auf eine separate Linse verzichtet werden. Ferner kann die beschriebene Einschnürung des von dem Endoskop erfassten Lichtbündels im Bereich von zumindest einem der beiden Prisma unterstützt werden.

Bei einem Endoskop, wie es hier beschrieben ist, kann ein mittlerer Abschnitt der optischen Achse zwischen dem schwenkbaren Prisma und dem feststehenden Prisma im Wesentlichen senkrecht zur Längsachse des Endoskops verlaufen.

Insbesondere kann zwischen der reflektierenden Fläche des schwenkbaren Prismas und der reflektierenden Fläche des feststehenden Prismas ein mittlerer Abschnitt der optischen Achse im Wesentlichen senkrecht zur Längsachse des Endoskops verlaufen.

Der mittlere Abschnitt der optischen Achse zwischen der reflektierenden Fläche des schwenkbaren Prismas und der reflektierenden Fläche des feststehenden Prismas ist die (insbesondere gerade) Linie, auf der ein Lichtstrahl verläuft, der proximal der reflektierenden Fläche des feststehenden Prismas auf der optischen Achse, insbesondere auf der optischen Achse nachfolgender Stablinsen, verläuft. Die optische Achse gibt insbesondere auch die Richtung des übertragenen Lichtbündels an. Wie bereits erwähnt, kann auch die Schwenkachse des schwenkbaren Prismas senkrecht zur Längsachse des Endoskops und damit insbesondere parallel zum mittleren Abschnitt der optischen Achse sein.

Insbesondere wenn sowohl der mittlere Abschnitt der optischen Achse zwischen den reflektierenden Flächen der Prismen als auch die Schwenkachse des schwenkbaren Prismas senkrecht zur Längsachse des Endoskops angeordnet ist, können die bereits erwähnten, besonders großen Blickrichtungsbereiche realisierbar sein.

Insbesondere bei der beschriebenen Einschnürung des von dem Endoskop erfassten Lichtbündels im Bereich des schwenkbaren Prismas bzw. im Bereich des feststehenden Prismas können mit den unterschiedlich großen reflektierenden Flächen ein besonders kleiner Abstand derselben und damit ein besonders kleiner seitlicher Versatz zwischen dem distalen Abschnitt der optischen Achse distal des schwenkbaren Prismas und dem proximalen Abschnitt der optischen Achse proximal des feststehenden Prismas realisierbar sein. Dadurch können bei einem gegebenen Querschnitt des Schafts des Endoskops besonders große Querschnitte von Stablinsen und besonders gute Helligkeit, Kontrast und Schärfe des erfassten Bilds erzielbar sein.

Ein Endoskop, wie es hier beschrieben ist, kann ferner eine Einrichtung zum Aufrichten eines mittels des Endoskops erfassten Bilds aufweisen.

Eine Einrichtung zum Aufrichten eines Bilds ist beispielsweise ein Prisma oder eine andere Einrichtung mit einer ungeraden Anzahl von reflektierenden Flächen, insbesondere ein Schmidt-Pechan-Prisma oder ein Dove-Prisma, alternativ möglicherweise ein Doppel-Dove-Prisma. Die Gesamtanzahl der reflektierenden Flächen des Endoskops wird dabei insbesondere gerade gewählt, um ein seitenverkehrtes Bild zu vermeiden. Die Einrichtung zum Aufrichten eines erfassten Bilds ist insbesondere am proximalen Ende des Endoskops angeordnet. Das Endoskop ist insbesondere so ausgebildet, dass beim Schwenken des schwenkbaren Prismas um seine Schwenkachse gleichzeitig die Einrichtung zum Aufrichten rotiert wird, insbesondere mit halber Rotationsgeschwindigkeit. Die Einrichtung zum Aufrichten wird dabei insbesondere um ihre optische Achse (distal ihrer ersten bzw. distalen reflektierenden Fläche und proximal der letzten bzw. proximalen reflektierenden Fläche) rotiert.

Die Einrichtung zum Aufrichten des mittels des Endoskops erfassten Bilds ermöglicht eine gleichbleibende Orientierung des mittels des Endoskops erfassen Bilds auch bei einer Schwenkbewegung des schwenkbaren Prismas um seine Schwenkachse.

Bei einem Verfahren zum Erfassen eines Bilds einen Gegenstands mittels eines Endoskops wird ein schwenkbares Prisma relativ zu dem Endoskop geschwenkt, um eine Blickrichtung des Endoskops einzustellen. Von dem Gegenstand ausgehendes Licht wird mittels des schwenkbaren Prismas umgelenkt, wobei vom schwenkbaren Prisma umgelenktes Licht zumindest entweder durch eine Lichtaustrittsfläche des schwenkbaren Prismas, die in einer Ausnehmung eines feststehenden Prismas angeordnet ist, aus dem schwenkbaren Prisma austritt oder durch eine Lichteintrittsfläche des feststehenden Prismas, die in einer Ausnehmung des schwenkbaren Prismas angeordnet ist, in das feststehende Prisma eintritt. Mittels des schwenkbaren Prismas umgelenktes Licht wird mittels eines feststehenden Prismas in eine Richtung umgelenkt, die im Wesentlichen parallel zur Längsachse des Schafts des Endoskops ist. Mittels des feststehenden Prismas umgelenktes Licht wird erfasst, um das Bild zu erfassen.

Das beschriebene Verfahren ist insbesondere mit Ausführungsformen des hier beschriebenen Endoskops durchführbar. Dabei sind die oben beschriebenen Vorteile erzielbar.

### Kurzbeschreibung der Figuren

Nachfolgend werden Ausführungsformen anhand der beigefügten Figuren näher erläutert. Es zeigen:
- Figur 1: eine schematische Darstellung eines Endoskops mit einstellbarer Blickrichtung;
- Figur 2: eine schematische Darstellung einer Ausführungsform des distalen Endes eines Endoskops;
- Figur 3: eine weitere schematische Darstellung der Ausführungsform aus Figur 2;
- Figur 4: eine weitere schematische Darstellung der Ausführungsform aus den Figuren 2 und 3;
- Figur 5: eine schematische Darstellung einer erfindungsgemäßen Ausführungsform des distalen Endes eines Endoskops;

- Figur 6: eine schematische Darstellung des Strahlengangs in der Ausführungsform aus Figur 5;
- Figur 7: schematische Darstellung einer weiteren erfindungsgemäßen Ausführungsform des distalen Endes eines Endoskops;
- Figur 8: schematische Darstellung einer ebenfalls erfindungsgemäßen Ausführungsform des distalen Endes eines Endoskops;

- Figur 9: ein schematisches Flussdiagramm eines Verfahrens zum Erfassen eines Bilds mittels eines Endoskops.

### Beschreibung von Ausführungsformen

Figur 1 zeigt eine schematische Darstellung eines Endoskops 10 mit einem distalen Ende 11, einem proximalen Ende 12 und einem starren Schaft 14, der sich vom distalen Ende 11 bis zum proximalen Ende 12 erstreckt. Alternativ ist der Schaft 14 flexibel oder teilweise flexibel. Der Querschnitt des Schafts 14 oder zumindest die äußere Kontur des Querschnitts des Schafts 14 ist zwischen dem distalen Ende 11 und dem proximalen Ende 12 konstant oder im Wesentlichen konstant. Insbesondere ist die Kontur des Querschnitts des Schafts 14 kreisförmig oder elliptisch. In diesem Fall ist die in Figur 1 dargestellt Längsachse 18 des Endoskops 10 die Symmetrieachse der Mantelfläche des Schafts 14 zwischen dem distalen Ende 12 und dem proximalen Ende 11. Bei einer kreiszylindrischen Mantelfläche des Schafts 14 ist die Längsachse 18 auch die Menge der Mittelpunkte oder Flächenschwerpunkte der Querschnitte des Schafts 14 zwischen dem distalen Ende 12 und dem proximalen Ende 11.

Am distalen Ende 12 weicht die Gestalt des Schafts 14 von der Zylindersymmetrie ab, wie dies beispielhaft in Figur 1 dargestellt ist. Insbesondere weist der Schaft 14 am distalen Ende 12 eine Öffnung auf, die durch ein transparentes Fensterbauteil mit einer gewölbten Oberfläche 20 verschlossen ist, insbesondere hermetisch dicht verschlossen ist. Die Oberfläche 20 des Fensterbauteils hat beispielsweise die Gestalt eines Ausschnitts eines Kreiszylindermantels, wobei die Symmetrieachse des Kreiszylinders senkrecht zur Längsachse 18 des Endoskops 10 und zur Zeichenebene der Figur 1 ist. Alternativ hat die Oberfläche 20 des transparenten Fensterbauteils die Gestalt eines Ausschnitts einer Kugeloberfläche oder eines zwei- oder dreiachsigen Ellipsoids.

Am distalen Enden 12 des Endoskops 10 sind im Schaft 14 optische Einrichtungen angeordnet, die in Figur 1 nicht dargestellt sind. Diese optischen Einrichtungen werden nachfolgend anhand der Figuren 2 bis 8 dargestellt und ermöglichen eine Variation der Blickrichtung des Endoskops zwischen einer ersten, extremen Blickrichtung 21 und einer vierten, extremen Blickrichtung 24. Eine zweite und eine dritte Blickrichtung, die zwischen der ersten Blickrichtung 21 und der vierten Blickrichtung 24 liegen, werden nachfolgend anhand der Figuren 3 und 4 dargestellt. Die Blickrichtung ist jeweils die Richtung bezogen auf das distale Ende 12 des Endoskops 10, in der ein Gegenstand liegt, der in der Mitte eines mittels des Endoskops 10 erfassten Bilds erscheint.

Bei dem in Figur 1 dargestellten Beispiel ist die erste, extreme Blickrichtung 21 parallel oder im Wesentlichen parallel zur Längsachse 18 des Endoskops 10. Zwischen den extremen Blickrichtungen 21, 24 liegt ein Winkelbereich 29, der bei dem dargestellten Beispiel ca. 120° umfasst. Innerhalb dieses Winkelbereichs ist die Blickrichtung des Endoskops 10 insbesondere kontinuierlich verstellbar bzw. einstellbar.

Am proximalen Ende 11 weist das Endoskop 10 eine erste Kupplung 32 zum optischen Koppeln des Endoskops 10 mit einer Kamera oder ein Okular sowie eine zweite Kupplung 34 zum Koppeln des Endoskops 10 mit einer Lichtquelle über ein Lichtleitkabel auf. Ferner weist das Endoskop 10 am proximalen Ende 11 eine Einrichtung 70 zum Aufrichten eines mittels des Endoskops 10 erfassten Bilds auf. Ohne die Einrichtung 70 hat bei dem anhand der Figuren 2 bis 8 dargestellten Ausführungsformen eine Variation der Blickrichtung in einer Ebene nicht nur eine Verschiebung des erfassten Bilds, sondern auch dessen Rotation zur Folge. Zur Kompensation der Rotation ist die Einrichtung 70 mit einer ungeraden Anzahl von reflektierenden Flächen vorgesehen. Mittels einer hier nicht näher beschriebenen Einrichtung wird sichergestellt, dass bei einer Variation der Blickrichtung des Endoskops 10 gleichzeitig die Einrichtung 70 um die Längsachse 18 des Endoskops oder um eine dazu parallele Achse so rotiert wird, dass das erfasste Bild nicht rotiert. Insbesondere ist eine Einrichtung vorgesehen, die bei einem Schwenken der nachfolgend anhand der Figuren 2 bis 8 dargestellten schwenkbaren Prismen um einen Winkel ein Rotieren der Einrichtung 70 um die Hälfte des Winkels bewirkt.

Die Figuren 2 bis 4 zeigen schematische Darstellungen des distalen Endes eines Endoskops mit einstellbarer Blickrichtung, insbesondere des oben anhand der Figur 1 dargestellten Endoskops 10. Die Zeichenebenen der Figuren 2 bis 4 sind jeweils parallel zur Längsachse 18 des Endoskops 10 und senkrecht zur Zeichenebene der Figur 1. Die Wandung des Schafts 14 ist jeweils nur schematisch durch eine rechteckige punktierte Linie angedeutet. Die Öffnung und das Fensterbauteil, die oben beschrieben sind, sind in den Figuren 2 bis 4 im Sinne der Übersichtlichkeit nicht dargestellt. Einrichtungen zum Übertragen bzw. Leiten von Beleuchtungslicht im Schaft 14, Einrichtungen zum Schwenken der nachfolgend beschriebenen schwenkbaren Prismen und weitere Details sind in den Figuren 2 bis 4 im Sinne der Übersichtlichkeit ebenfalls nicht dargestellt.

Das in den Figuren 2 bis 4 dargestellte Endoskop weist eine schwenkbare reflektierende Fläche 42 und eine feststehende reflektierende Fläche 52 auf, die bei dem dargestellten Beispiel jeweils eben sind. Die reflektierenden Flächen 42, 52 sind jeweils beispielhaft kreisförmig dargestellt.

Die schwenkbare reflektierende Fläche 42 ist um eine Schwenkachse 45 schwenkbar bzw. rotierbar. Die Schwenkachse 45 ist senkrecht zur Längsachse 18 des Endoskops, senkrecht zur Zeichenebene der Figur 1 und parallel zu den Zeichenebenen der Figuren 2 bis 4. Zwischen den Flächennormalen der schwenkbaren reflektierenden Fläche 42 und der feststehenden reflektierenden Fläche 52 einerseits und der Schwenkachse 45 der schwenkbaren reflektierenden Fläche 42 andererseits liegt jeweils ein Winkel von ca. 45°. Die Flächennormale der feststehenden reflektierenden Fläche 52 liegt parallel zu den Zeichenebenen der Figuren 2 bis 4.

Proximal schließen sich an die feststehende reflektierende Fläche 52 eine Linse 61 und mehrere Stablinsen 64 zum Übertragen von Licht zum proximalen Ende des Endoskops an. Anstelle einer einzelnen Linse 61 kann eine Gruppe von Linsen oder ein Objektiv vorgesehen sein.

Die Figuren 2, 3 und 4 zeigen die schwenkbare reflektierende Fläche 42 in drei verschiedenen Winkelpositionen entsprechend drei verschiedenen Blickrichtungen 21, 22 bzw. 23. Bei der in Figur 2 dargestellten Orientierung der schwenkbaren reflektierenden Fläche 42 ist die Flächennormale der schwenkbaren reflektierenden Fläche 42 parallel zur Zeichenebene der Figur 2. Die Blickrichtung entspricht der anhand der Figur 1 dargestellten ersten, extremen Blickrichtung 21 parallel zur Längsachse 18 des Endoskops 10.

In Figur 3 ist die schwenkbare reflektierende Fläche 42 in einer Orientierung dargestellt, die gegenüber der oben anhand der Figur 2 dargestellten Orientierung um 45° um die Schwenkachse 45 rotiert ist. Die damit realisierte zweite Blickrichtung 22 schließt sowohl mit der Längsachse 18 des Endoskops 10 als auch mit der Normalen der Zeichenebene der Figur 3 jeweils einen Winkel von 45° ein.

Bei der in Figur 4 dargestellten Orientierung der reflektierenden Fläche 42 ist diese gegenüber der oben anhand der Figur 2 dargestellten Orientierung um 90° und gegenüber der oben anhand der Figur 3 dargestellten Orientierung um weitere 45° um die Schwenkachse 45 rotiert. Die resultierende dritte Blickrichtung 23 ist senkrecht zur Zeichenebene der Figur 4.

In den Figuren 2 und 3 ist ferner jeweils der distale Abschnitt 48 der optischen Achse distal der schwenkbaren reflektierenden Fläche 42 dargestellt. In jeder der Figuren 2 bis 4 sind ferner der mittlere Abschnitt 58 der optischen Achse zwischen den reflektierenden Flächen 42, 52 und der proximale Abschnitt 68 der optischen Achse proximal der feststehenden reflektierenden Fläche 52 dargestellt. Der proximale Abschnitt 68 der optischen Achse proximal der feststehenden reflektierenden Fläche 52 ist bei diesem Beispiel gleichzeitig die optische Achse der Linse 61 und die optische Achse der Stablinsen 64. Die optischen Achsen der Linse 61 und der Stablinsen 64 sind insbesondere Symmetrieachsen, bezüglich derer die Linse 61 bzw. die Stablinsen 64 eine Rotationssymmetrie aufweisen.

Im Bild der geometrischen bzw. Strahlenoptik verläuft ein von einem Gegenstand, der in der Blickrichtung 21, 22, 23 liegt, ausgehender Lichtstrahl, der proximal der feststehenden reflektierenden Fläche 52 entlang dem proximalen Abschnitt 68 der optischen Achse verläuft, distal der schwenkbaren reflektierenden Fläche 42 auf dem distalen Abschnitt 48 der optischen Achse und zwischen den reflektierenden Flächen 42, 52 auf dem proximalen Abschnitt 48 der optischen Achse 58. Somit schneiden sich der distale Abschnitt 48 und der mittlere Abschnitt 58 der optischen Achse in einem Punkt, insbesondere im Mittelpunkt, der schwenkbaren reflektierenden Fläche 42 und der mittlere Abschnitt 58 und der proximale Abschnitt 68 der optischen Achse in einem Punkt, insbesondere im Mittelpunkt, der feststehenden reflektierenden Fläche 52.

Aus der Blickrichtung 21, 22 bzw. 23 oder aus einem vorbestimmten Raumwinkelbereich um diese Blickrichtung 21, 22 bzw. 23 auf die schwenkbare reflektierende Fläche 42 fallendes Licht wird teilweise, überwiegend oder vollständig zur feststehenden reflektierenden Fläche 52 umgelenkt. Von der feststehenden reflektierenden Fläche 52 wird das Licht teilweise oder vollständig zur Linse 61 umgelenkt und durch die Linse 61 und die Stablinsen 64 zum proximalen Ende 11 des Endoskops 10 übertragen, um dort beispielsweise in einer Kamera oder auf der Netzhaut eines menschlichen Auges an einem Okular ein reales Bild des Gegenstands zu erzeugen, von dem das Licht ausgeht. Der vorbestimmte Raumwinkelbereich, in dem Gegenstände vom distalen Ende 12 des Endoskops aus gesehen liegen, von denen durch das Endoskop 10 an dessen proximalem Ende 11 ein Bild erzeugt wird, wird auch als Sichtfeld bezeichnet.

In der Zusammenschau der Figuren 2 bis 4 ist erkennbar, dass die Blickrichtung 21, 22, 23 des Endoskops 10 durch Schwenken der reflektierenden Flächen 42 um die Schwenkachse 45 in einem großen Winkelbereich 29 eingestellt werden kann. Die Größe des Schwenkbereichs der schwenkbaren reflektierenden Fläche 42 und die entsprechende Größe des Winkelbereichs 29, innerhalb dessen die Blickrichtung 21, 22, 23, 24 einstellbar ist, ist bei der in den Figuren 2 bis 4 dargestellten Ausführungsform nicht oder nicht wesentlich durch die optischen Elemente 42, 52, 61 eingeschränkt. Bei einer entsprechenden Ausgestaltung des distalen Endes 12 des Endoskops 10 und insbesondere der dort angeordneten und durch ein transparentes Fensterbauteil verschlossenen Öffnung, sind Blickrichtungen in Winkelbereichen realisierbar, die von der Blickrichtung 21 parallel zur Längsachse 18 des Endoskops 10 ausgehend in einer Richtung oder in zwei entgegengesetzten Richtungen ohne Weiteres mehr als 90° oder sogar 120° oder mehr umfassen können. Gleichzeitig können Sichtfelder mit einem Winkel von 70° oder mehr realisierbar sein.

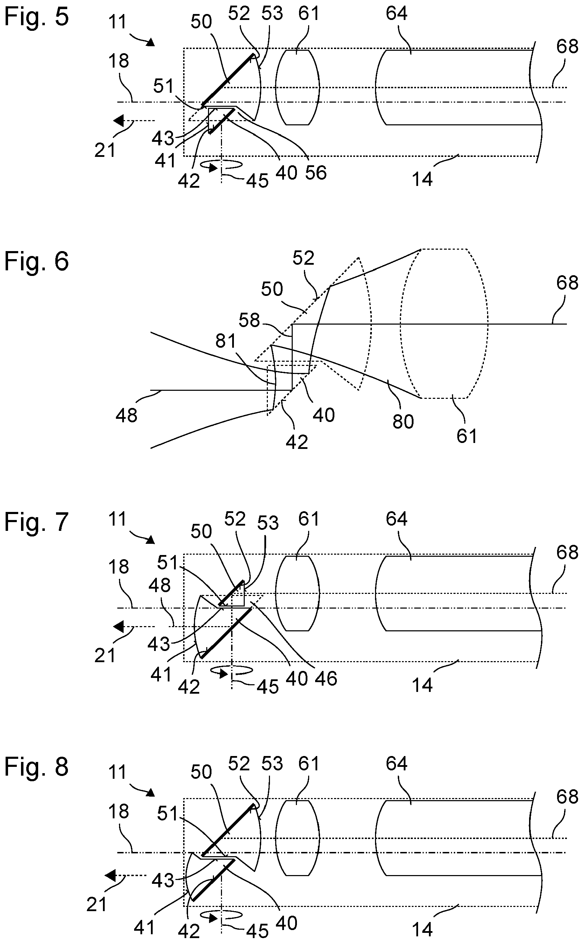

Die Figuren 5 bis 8 zeigen schematische Darstellungen des erfindungsgemäßen distalen Endes eines Endoskops mit einstellbarer Blickrichtung, insbesondere des oben anhand der Figur 1 dargestellten Endoskops 10. Die Zeichenebenen der Figuren 5 bis 8 entsprechen den Zeichenebenen der Figuren 2 bis 4, sind also wiederum parallel zur Längsachse 18 des Endoskops 10 und senkrecht zur Zeichenebene der Figur 1. Auch hinsichtlich der auf das für die nachfolgenden Ausführungen Wesentliche reduzierten Darstellung des Schafts 14 (insbesondere ohne Öffnung und Fensterbauteil) und des Verzichts auf die Darstellung von Einrichtungen zur Erzeugung oder Übertragung von Beleuchtungslicht sowie von mechanischen Einrichtungen zum Schwenken der nachfolgend beschriebenen Prismen entsprechen die Figuren 5 bis 8 den Figuren 2 bis 4.

Im Unterschied zu den Figuren 2 bis 4 ist in den Figuren 5 bis 8 jedoch jeweils nur die erste, extreme Blickrichtung 21 dargestellt. Auf eine Darstellung der nachfolgend beschriebenen schwenkbaren Prismen in Orientierungen, die anderen Blickrichtungen entsprechen, wurde im Sinne einer kompakten Beschreibung verzichtet. Für den Fachmann ist jedoch offensichtlich, wie die nachfolgend beschriebenen schwenkbaren Prismen um deren Schwenkachsen geschwenkt werden können, um andere Blickrichtungen einzustellen. Insbesondere sind die Prismen in entsprechender Weise schwenkbar wie oben anhand der Figuren 2 bis 4 für die reflektierende Fläche 42 dargestellt.

Bei den in den Figuren 5 bis 8 dargestellten Ausführungsformen sind die reflektierenden Flächen 42, 52 jeweils Bestandteil eines schwenkbaren Prismas 40 bzw. eines feststehenden Prismas 50. Bei jeder der in den Figuren 5 bis 8 dargestellten Ausführungsformen weist das schwenkbare Prisma 40 ferner eine Lichteintrittsfläche 41 und eine Lichtaustrittsfläche 43 auf. Bei jeder der in den Figuren 5 bis 8 dargestellten Ausführungsformen weist das feststehende Prisma 50 ferner eine Lichteintrittsfläche 51 und eine Lichtaustrittsfläche 53 auf.

Die Lichteintrittsfläche 41 des schwenkbaren Prismas 40 ist jeweils der Blickrichtung 21 zugewandt, wobei insbesondere zumindest auf dem distalen Abschnitt 48 der optischen Achse die Flächennormale der Lichteintrittsfläche 41 parallel zur Blickrichtung 21 ist. Die Lichtaustrittsfläche 43 des schwenkbaren Prismas 40 und die Lichteintrittsfläche 51 des feststehenden Prismas 50 sind einander zugewandt, wobei insbesondere zumindest auf dem mittleren Abschnitt 58 der optischen Achse zwischen den reflektierenden Flächen 42, 52 die Flächennormalen der Lichtaustrittsfläche des schwenkbaren Prismas 43 und der Lichteintrittsfläche 51 des feststehenden Prismas 50 antiparallel zueinander sind. Die Lichtaustrittsfläche 53 des feststehenden Prismas 53 ist jeweils der Linse 61 zugewandt, wobei insbesondere zumindest auf dem proximalen Abschnitt 68 der optischen Achse die Flächennormale der Lichtaustrittsfläche 53 parallel zum proximalen Abschnitt 68 der optischen Achse ist.

Die in den Figuren 5 bis 8 dargestellten Ausführungsformen unterscheiden sich hinsichtlich der Größen und der Gestalten der schwenkbaren Prismen 40 und der feststehenden Prismen 50.

Bei der in Figur 5 dargestellten Ausführungsform ist die reflektierende Fläche 52 des feststehenden Prismas 50 größer als die reflektierende Fläche 42 des schwenkbaren Prismas 40. Insbesondere verhalten sich die linearen Abmessungen oder die Flächeninhalte der reflektierenden Fläche 52 des feststehenden Prismas 50 und der reflektierenden Fläche 42 des schwenkbaren Prismas 40 mindestens wie 4:3 oder mindestens wie 3:2 oder mindestens wie 2:1. Alternativ können beide reflektierende Flächen 42, 52 abweichend von der Darstellung in Figur 5 gleich groß oder im Wesentlichen gleich groß sein.

Ferner ist die Lichtaustrittsfläche 53 des feststehenden Prismas 50 gekrümmt, insbesondere konvex gekrümmt. Alternativ kann die Lichtaustrittsfläche 53 des feststehenden Prismas 50 abweichend von der Darstellung in Figur 5 eben sein.

Ferner ist das schwenkbare Prisma 40 zumindest teilweise in einer Ausnehmung 56 des feststehenden Prismas 50 angeordnet. Insbesondere ist die Lichtaustrittsfläche 43 des schwenkbaren Prismas in der Ausnehmung 56 des feststehenden Prismas 50 angeordnet. Die Ausnehmung 56 ist vor allem im Vergleich zur in einer unterbrochenen Linie dargestellten Kontur eines feststehenden Prismas 50 ohne Ausnehmung gut erkennbar. Die Lichteintrittsfläche 51 des feststehenden Prismas 50 bildet einen innerhalb der Ausnehmung 56 liegenden Teilbereich der Oberfläche des feststehenden Prismas 50.

Der in der Ausnehmung 56 liegende Teilbereich der Oberfläche des feststehenden Prismas 50 ist insgesamt konkav oder im Wesentlichen konkav und umfasst ebene Unterbereiche. Insbesondere ist die Lichteintrittfläche 51 des feststehenden Prismas 50 eben. Alternativ kann der in der Ausnehmung 56 liegende Teilbereich der Oberfläche des feststehenden Prismas 50 lokal konvexe Unterbereiche umfassen. Beispielsweise kann die Lichteintrittfläche 51 des feststehenden Prismas 50 abweichen von der Darstellung in Figur 5 konvex sein. Die insgesamt konkave Eigenschaft des in der Ausnehmung 56 liegenden Teilbereichs der Oberfläche des feststehenden Prismas 50 ist insbesondere in der in Figur 5 dargestellten Schnittebene erkennbar.

Bei dem in Figur 5 dargestellten Beispiel ist die Ausnehmung 56 ferner dadurch charakterisiert, dass ein Teil des feststehenden Prismas 50, insbesondere ein Teil der Lichtaustrittfläche 53 des feststehenden Prismas 50, gegenüber einer Ebene, in der die Lichteintrittfläche 51 liegt, übersteht bzw. über diese Ebene hinaus ragt.

Figur 6 zeigt eine vergrößerte Darstellung eines Ausschnitts der in Figur 5 dargestellten Ausführungsform. Die Prismen 40, 50 und die Linse 61 sind lediglich in unterbrochener Linie angedeutet. In durchgezogenen Linien ist ein Lichtbündel 80 aus dem Sichtfeld dargestellt. Das Lichtbündel 80 wird mittels der reflektierenden Flächen 42, 52 des schwenkbaren Prismas 40 bzw. des feststehenden Prismas 50 zur Linse 61 umgelenkt. Die Prismen 40, 50 und die Linse 61 sowie ggf. eine oder mehrere in den Figuren 5 und 6 nicht dargestellte Linsen distal des schwenkbaren Prismas 40 sind so ausgebildet, dass das Lichtbündel 80 im Bereich des schwenkbaren Prismas 40 eine Taille bzw. Einschnürung 81 aufweist. Diese Einschnürung 81 des Lichtbündels 80 kann dazu beitragen, einen ausgeprägten Größenunterschied zwischen der reflektierenden Fläche 42 des schwenkbaren Prismas 40 einerseits und der reflektierenden Fläche 52 des feststehenden Prismas und der Querschnittsfläche des Lichtbündels 80 proximal des feststehenden Prismas 50 andererseits zu ermöglichen.

Figur 7 zeigt eine schematische Darstellung einer Ausführungsform, die sich von der oben anhand der Figur 5 dargestellten Ausführungsform unter anderem darin unterscheidet, dass das schwenkbare Prisma 40 und die reflektierende Fläche 42 des schwenkbaren Prismas 40 größer oder deutlich größer als das feststehende Prisma 50 bzw. dessen reflektierende Fläche 52 sind.

Ferner unterscheidet sich die in Figur 7 dargestellte Ausführungsform von der oben anhand der Figur 5 dargestellten Ausführungsform darin, dass das feststehende Prisma 50 teilweise in einer Ausnehmung 46 des schwenkbaren Prismas 40 angeordnet ist. Insbesondere ist die Lichteintrittsfläche 51 des feststehenden Prismas 50 in der Ausnehmung 46 des schwenkbaren Prismas 40 angeordnet. Die Lichtaustrittsfläche 43 des schwenkbaren Prismas 40 bildet einen Teilbereich der Oberfläche des schwenkbaren Prismas 40 in der Ausnehmung 46. Die Ausnehmung 46 ist vor allem im Vergleich zur in unterbrochener Linie dargestellten Kontur eines schwenkbaren Prismas ohne Ausnehmung erkennbar.

Ferner unterscheidet sich die in Figur 7 dargestellte Ausführungsform von der oben anhand der Figur 5 dargestellten Ausführungsform dadurch, dass die Lichteintrittsfläche 41 des schwenkbaren Prismas 40 gekrümmt, insbesondere konvex, ist. Alternativ kann die Lichteintrittsfläche 41 des schwenkbaren Prismas 40 abweichend von der Darstellung in Figur 7 eben oder konkav sein.

Bei der in Figur 7 dargestellten Ausführungsform weist ein von den reflektierenden Flächen 42, 52 der Prismen 40, 50 auf die Linse 61 gelenktes Lichtbündel im Bereich des feststehenden Prismas 50 eine Taille bzw. Einschnürung auf. Das Lichtbündel und dessen Einschnürung sind in Figur 7 nicht dargestellt, sind jedoch den in Figur 6 dargestellten ähnlich.

Figur 8 zeigt eine schematische Darstellung einer Ausführungsform, die sich von den oben anhand der Figuren 5 bis 7 dargestellten Ausführungsformen dadurch unterscheidet, dass sowohl das schwenkbare Prisma 40 als auch das feststehende Prisma 50 jeweils eine Ausnehmung aufweisen. Da diese Ausnehmungen nach den obigen Darstellungen anhand der Figuren 5 bis 7 auch in Figur 8 für Fachleute klar erkennbar sind, wurde auf deren Kennzeichnung im Sinne der Übersichtlichkeit verzichtet.

Ferner unterscheidet sich die in Figur 8 dargestellte Ausführungsform von den anhand der Figuren 5 bis 7 dargestellten Ausführungsformen dadurch, dass sowohl die Lichteintrittsfläche 41 des schwenkbaren Prismas 40 als auch die Lichtaustrittsfläche 53 des feststehenden Prismas 50 jeweils konvex gekrümmt bzw. gewölbt sind. Alternativ können die Lichteintrittsfläche 41 des schwenkbaren Prismas 40 und die Lichtaustrittsfläche 53 des feststehenden Prismas 50 jeweils eben oder konkav gekrümmt sein.

Das schwenkbare Prisma 40 ist bei der anhand der Figur 8 dargestellten Ausführungsform kleiner als das feststehende Prisma 50. Alternativ können abweichend von der Darstellung in Figur 8 beide Prismen 40, 50 gleich groß oder das schwenkbare Prisma 40 größer als das feststehende Prisma 50 sein.

Abweichend von den Darstellungen in den Figuren 2 bis 8 können auch bei den anhand der Figuren 2 bis 8 dargestellten Ausführungsformen die reflektierenden Flächen 42, 52 konvex oder konkav gekrümmt sein. Ferner können auch die in den Figuren 5 bis 8 eben dargestellten Lichteintrittsflächen 41, 51 und Lichtaustrittsflächen 43, 53 konvex oder konkav gekrümmt sein. Durch gekrümmte Lichteintrittsflächen 41, 51, gekrümmte reflektierende Flächen 42, 52 und gekrümmte Lichtaustrittsflächen 43, 53 können die Prismen 40, 50 nicht nur lichtumlenkende Eigenschaften, sondern gleichzeitig abbildende Eigenschaften (insbesondere Erzeugung reeller oder virtueller Bilder) aufweisen und die oben anhand der Figuren 6 bis 8 und insbesondere anhand der Figur 7 dargestellte Einschnürung des Lichtbündels unterstützen. Ferner kann sowohl das schwenkbare Prisma 40 als auch das feststehende Prisma 50 jeweils mehrere reflektierende Flächen aufweisen, beispielsweise in Form eines Pentaprismas oder eines Dachkant-Pentaprismas.

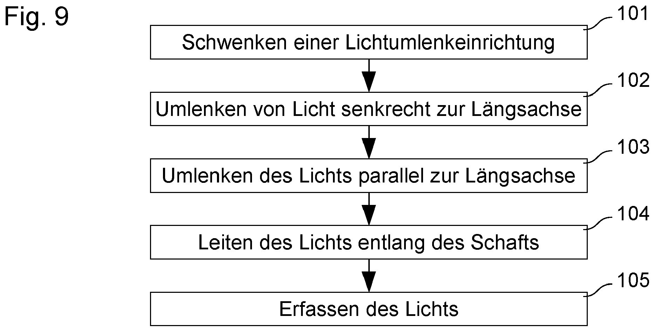

Figur 9 zeigt ein schematisches Flussdiagramm eines Verfahrens zum Erfassen eines Bilds eines Gegenstands mittels eines Endoskops. Obwohl dieses Verfahren auch mit einem Endoskop durchführbar ist, das sich von den oben anhand der Figuren 1 bis 8 dargestellten Ausführungsformen unterscheidet, werden nachfolgend beispielhaft Bezugszeichen aus den Figuren 1 bis 8 verwendet, um das Verständnis zu erleichtern.

Bei einem ersten Schritt 101 wird ein schwenkbares Prisma 40, 42 geschwenkt, um eine Blickrichtung des Endoskops 10 einzustellen. Das Prisma wird um eine Schwenkachse 45 geschwenkt, die insbesondere senkrecht zur Längsachse des Endoskops ist. Die Blickrichtung wird insbesondere auf einen zu beobachtenden Gegenstand oder im Wesentlichen auf den zu beobachtenden Gegenstand eingestellt. Die nachfolgend beschriebenen Schritte können gleichzeitig mit dem ersten Schritt 101 ausgeführt werden.

Bei einem zweiten Schritt 102 wird Licht aus einer durch Schwenken des schwenkbaren Prismas 40 eingestellten Blickrichtung 21, 22, 23, 24 in eine Richtung im Wesentlichen parallel zur Schwenkachse 45 umgelenkt. Dies erfolgt insbesondere mittels einer reflektierenden Fläche 42 des Prismas 40. Vom schwenkbaren Prisma (40) umgelenktes Licht tritt durch eine Lichtaustrittsfläche (43) des schwenkbaren Prismas (40), die in einer Ausnehmung (56) eines feststehenden Prismas (50) angeordnet ist, aus dem schwenkbaren Prisma (40) aus. Alternativ oder zusätzlich tritt das vom schwenkbaren Prisma (40) umgelenkte Licht durch eine Lichteintrittsfläche (51) des feststehenden Prismas (50), die in einer Ausnehmung (46) des schwenkbaren Prismas (40) angeordnet ist, in das feststehende Prisma (50) ein.

Bei einem dritten Schritt 103 wird Licht, das sich im Wesentlichen parallel zur Schwenkachse 45 des schwenkbaren Prismas 40 ausbreitet, in eine Richtung, die im Wesentlichen parallel zur Längsachse 18 des Endoskops 10 ist, umgelenkt. Dies erfolgt insbesondere mittels einer reflektierenden Fläche 52 des Prismas 50. Bei einem optionalen vierten Schritt 104 wird beim zweiten Schritt 102 und beim dritten Schritt 103 umgelenktes Licht entlang des Schafts 14 des Endoskops 10 zum proximalen Ende 11 des Endoskops 10 geleitet. Bei einem fünften Schritt 105 wird beim zweiten Schritt 102 und beim dritten Schritt 103 umgelenktes und optional beim vierten Schritt 104 zum proximalen Ende 11 des Endoskops 10 geleitetes Licht erfasst. Dabei wird mittels einer Kamera oder mittels eines Okulars und des menschlichen Auges ein Bild des Gegenstands, von dem das Licht ausgeht, erfasst.

### Bezugszeichen

- 10: Endoskop
- 11: distales Ende des Endoskops 10
- 12: proximales Ende des Endoskops 10
- 14: Schaft des Endoskops 10
- 18: Längsachse des Endoskops 10
- 20: Oberfläche eines Fensterbauteils
- 21: erste, extreme Blickrichtung (0°)
- 22: zweite Blickrichtung (45°)
- 23: dritte Blickrichtung (90°)
- 24: vierte, extreme Blickrichtung (110°)
- 29: Winkelbereich der Blickrichtungen
- 32: erste Kupplung
- 34: zweite Kupplung
- 40: schwenkbares Prisma
- 41: Lichteintrittsfläche des schwenkbaren Prismas 40
- 42: reflektierende Fläche (des schwenkbaren Prismas 40)
- 43: Lichtaustrittsfläche des schwenkbaren Prismas 40
- 45: Schwenkachse des schwenkbaren Prismas 40
- 46: Ausnehmung im schwenkbaren Prisma 40
- 48: distaler Abschnitt der optischen Achse distal der reflektierenden Fläche 42
- 50: feststehendes Prisma
- 51: Lichteintrittsfläche des feststehendes Prismas 50
- 52: reflektierende Fläche (des feststehendes Prismas 50)
- 53: Lichtaustrittsfläche des feststehendes Prismas 50
- 56: Ausnehmung im feststehenden Prisma 50
- 58: mittlerer Abschnitt der optischen Achse zwischen den reflektierenden Flächen 42, 52
- 61: Linse
- 64: Stablinse im Schaft 14
- 68: proximaler Abschnitt der optischen Achse proximal der reflektierenden Fläche 52
- 70: Einrichtung zum Aufrichten eines Bilds
- 80: Lichtbündel
- 81: Einschnürung des Lichtbündels 80

- 101: erster Schritt
- 102: zweiter Schritt
- 103: dritter Schritt
- 104: vierter Schritt
- 105: fünfter Schritt

## Patentansprüche

1. Endoskop (10) mit einstellbarer Blickrichtung (21, 22, 23, 24), mit:
einem schwenkbaren Prisma (40) zum Umlenken von Licht, das zum Einstellen der Blickrichtung (21, 22, 23, 24) um eine Schwenkachse (45) schwenkbar ist;
einem feststehenden Prisma (50) zum Umlenken von Licht, das von dem schwenkbaren Prisma (40) umgelenkt ist, in eine Richtung parallel zur Längsachse (18) des Endoskops (10),
wobei zumindest entweder eine Lichtaustrittsfläche (43) des schwenkbaren Prismas (40) in einer Ausnehmung (56) des feststehenden Prismas (50) oder eine Lichteintrittsfläche (51) des feststehenden Prismas (50) in einer Ausnehmung (46) des schwenkbaren Prismas (40) angeordnet ist,
wobei ein von dem Endoskop (10) erfasstes Lichtbündel (80) zumindest entweder im Bereich des schwenkbaren Prismas (40) oder im Bereich des feststehenden Prismas (50) eine Einschnürung (81) aufweist und
zumindest entweder die Lichteintrittsfläche (41) des schwenkbaren Prismas (40) oder die Lichtaustrittfläche (53) des feststehenden Prismas (50) gekrümmt ist.

2. Endoskop (10) nach dem vorangehenden Anspruch, bei dem eine reflektierende Fläche (52) des feststehenden Prismas (50) größer als eine reflektierende Fläche (42) des schwenkbaren Prismas (40) ist.

3. Endoskop (10) nach Anspruch 1, bei dem eine reflektierende Fläche (42) des schwenkbaren Prismas (40) größer als eine reflektierende Fläche (52) des feststehenden Prismas (50) ist.

4. Endoskop (10) nach einem der vorangehenden Ansprüche, bei dem zumindest entweder das schwenkbare Prisma (40) oder das feststehende Prisma (50) eine abbildende Eigenschaft aufweist.

5. Endoskop (10) nach einem der vorangehenden Ansprüche, bei dem ein mittlerer Abschnitt der optischen Achse (58) zwischen dem schwenkbaren Prisma (40) und dem feststehenden Prisma (50) im wesentlichen senkrecht zur Längsachse (18) des Endoskops (10) verläuft.

## Claims

1. Endoscope (10) with an adjustable viewing direction (21, 22, 23, 24), comprising:
a pivotable prism (40) for deflecting light, said prism being pivotable about a pivot axis (45) for the purposes of setting the viewing direction (21, 22, 23, 24);
a stationary prism (50) for deflecting light into a direction parallel to the longitudinal axis (18) of the endoscope (10), said light having been deflected by the pivotable prism (40),
wherein at least either a light exit surface (43) of the pivotable prism (40) is disposed in a cutout (56) of the stationary prism (50) or a light entry surface (51) of the stationary prism (50) is disposed in a cutout (46) of the pivotable prism (40),
wherein a light beam (80) captured by the endoscope (10) has a waist (81) at least either in the region of the pivotable prism (40) or in the region of the stationary prism (50) and
at least either the light entry surface (41) of the pivotable prism (40) or the light exit surface (53) of the stationary prism (50) is curved.

2. Endoscope (10) according to the preceding claim, wherein a reflecting area (52) of the stationary prism (50) is greater than a reflecting area (42) of the pivotable prism (40).

3. Endoscope (10) according to Claim 1, wherein a reflecting area (42) of the pivotable prism (40) is greater than a reflecting area (52) of the stationary prism (50).

4. Endoscope (10) according to any one of the preceding claims, wherein at least either the pivotable prism (40) or the stationary prism (50) has an imaging property.

5. Endoscope (10) according to any one of the preceding claims, wherein a mid section of the optical axis (58) between the pivotable prism (40) and the stationary prism (50) extends substantially perpendicular to the longitudinal axis (18) of the endoscope (10).

## Revendications

1. Endoscope (10) à direction d'observation réglable (21, 22, 23, 24), comprenant :
un prisme pivotant (40) servant à dévier la lumière, qui peut pivoter autour d'un axe de pivotement (45) pour régler la direction d'observation (21, 22, 23, 24) ;
un prisme fixe (50) servant à dévier la lumière déviée par le prisme pivotant (40) dans une direction parallèle à l'axe longitudinal (18) de l'endoscope (10),
dans lequel au moins soit une surface de sortie de lumière (43) du prisme pivotant (40) est disposée dans un évidement (56) du prisme fixe (50), soit une surface d'entrée de lumière (51) du prisme fixe (50) est disposée dans un évidement (46) du prisme pivotant (40),
dans lequel un faisceau lumineux (80) détecté par l'endoscope (10) présente un rétrécissement (81) au moins soit dans la zone du prisme pivotant (40), soit dans la zone du prisme fixe (50) et
au moins soit la surface d'entrée de lumière (41) du prisme pivotant (40), soit la surface de sortie de lumière (53) du prisme fixe (50) est incurvée.

2. Endoscope (10) selon la revendication précédente, dans lequel une surface réfléchissante (52) du prisme fixe (50) est plus grande qu'une surface réfléchissante (42) du prisme pivotant (40).

3. Endoscope (10) selon la revendication 1, dans lequel une surface réfléchissante (42) du prisme pivotant (40) est plus grande qu'une surface réfléchissante (52) du prisme fixe (50).

4. Endoscope (10) selon l'une des revendications précédentes, dans lequel au moins soit le prisme pivotant (40), soit le prisme fixe (50) présente une propriété de formation d'image.

5. Endoscope (10) selon l'une des revendications précédentes, dans lequel une partie centrale de l'axe optique (58) entre le prisme pivotant (40) et le prisme fixe (50) est sensiblement perpendiculaire à l'axe longitudinal (18) de l'endoscope (10).
